# EUROPEAN PATENT SPECIFICATION

(11) **EP 0 716 067 B1**
(45) Date of publication and mention of the grant of the patent: **20.09.2000**
(21) Application number: 95308746.7
(22) Date of filing: 05.12.1995
(51) Int. Cl.: C07C 29/141, C07H 3/02, C07C 31/18

(54) **Process for the production of xylitol**
Verfahren zur Herstellung von Xylitol
Procédé pour la préparation de xylitol

(30) Priority: 06.12.1994 GB 9424567
(43) Date of publication of application: 12.06.1996
(73) Proprietor: CERESTAR HOLDING BV, NL-4550 AA Sas Van Gent (NL)
(72) Inventor: Beck, Roland Herwig Friedrich, Dr., B-3078 Everberg (BE); Elseviers, Myriam, B-1910 Kampenhout (BE); Coomans, Sonia Marianne Jeannine, B-1800 Vilvoorde (BE)
(74) Representative: Wilkinson, Stephen John

(56) References cited:
- WO-A-93/19030
- US-A- 2 868 847
- US-A- 3 755 294
- US-A- 4 156 076
- US-A- 4 355 158
- BERICHTE DER DEUTSCHEN CHEMISCHEN GESELLSCHAFT, vol. 32, 27 February 1899 WEINHEIM, DE, pages 550-560, O. RUFF: 'd- und r-Arabinose'
- JOURNAL OF THE AMERICAN CHEMICAL SOCIETY, vol. 56, no. 7, 5 July 1934 WASHINGTON, DC, US, pages 1632-1633, R.C. HOCKETT, ET AL.: 'Improvements in the preparation of d-arabinose from calcium gluconate'
- JOURNAL OF THE AMERICAN CHEMICAL SOCIETY, vol. 81, no. 19, 16 October 1959 WASHINGTON, DC, US, pages 5190-5192, R.L. WHISTLER, ET AL.: 'Preparation of D-arabinose from D-glucose with hypochlorite'

## Description

The present invention relates to a method for producing a pentitol from a hexaldonic acid. Specifically, the present invention relates to a method for producing xylitol starting from gluconic acid.

At present xylitol is mainly produced on industrial scale by hydrogenation of xylose. Xylose is obtained from xylan for example through hydrolysis. Xylan containing sources such as almond shells, corn cobs or birch wood are used as starting materials. The hydrolysis process suffers from the disadvantages of low yield and low product purity. Extensive purification has to be performed, i.e. ion-exchange treatment to remove the acid used for hydrolysis and colour, and crystallisation to remove the other hemicellulosic sugars also formed during hydrolysis. The purification is required to make the product suitable for application in food.

The processing of 12-13 kg almond shells is required to obtain 1 kg of crystalline xylitol resulting in about 11-12 kg of solid waste.

There is therefore a need for a xylitol production process wherein the starting material is readily available and wherein the amount of waste product formed is reduced.

Recently, methods have been described wherein xylitol is produced starting from other hexoses. In particular D-glucose, D-galactose, D-fructose or L-sorbose were used as starting material. In a first step the hexose is submitted to a chain shortening reaction which yields a *C*_{*5*}*-intermediate.* The second basic reaction step (which may be actually more than one step) is the conversion of the *C*_{*5*}*-intermediate* into xylitol.

European patent application EP 403 392 and EP 421 882 (both Roquette Frères) disclose a process in which glucose is fermented to D-arabinitol by an osmophilic yeast. Subsequently, the arabinitol *(C*_{*5*}*-intermediate)* is converted by bacteria (Acetobacter, Gluconobacter or Klebsiella) into D-xylulose. The xylulose is then isomerised by glucose (xylose) isomerase into a xylose/xylulose mixture and either directly hydrogenated or hydrogenated after prior enrichment of xylose.

In International patent application WO 93/19030 (Amylum) glucose, fructose, galactose or mixtures thereof (obtained through hydrolysis of the disaccharides sucrose and lactose) are oxidatively decarboxylated to give alkali arabinonate and lyxonate, respectively. These aldonic acids are the *C*_{*5*}*-intermediates,* which are transformed into xylitol via arabinitol (=lyxitol). When L-sorbose is used as a starting material in this process L-xylonate is obtained, which can be directly hydrogenated to xylitol.

Other well known chemical methods for xylitol production include protection group chemistry. Due to their lack of commercial interest they are not considered in detail here (Helv.Chim.Acta 58, 1975, 311).

Several purely microbiological pathways have also been published. However none of them can compete economically because the overall yield is too low.

US4156076 discloses a process in which a lactose solution is oxidatively hydrolysed to form galactose and gluconic acid. The gluconic acid and galactose may be separated and converted to useful end products, for example gluconic acid may be oxidatively decarboxylated to arabinose.

US3755294 describes a process for the production of arabinose which involves the reaction of calcium gluconate with hydrogen peroxide in the presence of a catalytic amount of ferric gluconate.

There exists therefore a need for an economically valuable method of producing pentitols, especially xylitol, with a low level of impurities, comprising a reaction sequence giving easily obtainable intermediates, and which are easily refined according to methods known in the art.

The present invention provides such a method. The present invention relates to a method for producing xylitol from gluconic 'acid, whereby the acid is free, or in the form of its salt or the corresponding lactone. The method comprises the following steps:
a. decarboxylation of gluconic acid, performed using a (i) hypochlorite or (ii) hydrogen peroxide in the presence of a catalytic amount of Fe³⁺ or Cu²⁺, wherein in both (i) and (ii) the pH is kept substantially constant, to give an intermediate consisting mainly of arabinose,
b. hydrogenation of arabinose in the presence of a catalyst to give the corresponding pentitol; arabinitol,
c. catalytic isomerisation of the arabinitol to a pentitol mixture,
d. separation of xylitol from the pentitol mixture, and
e. optionally recycling of the other pentitols, mainly arabinitol and ribitol, to the isomerisation step (c).

Starting with gluconic acid the method of the present invention gives a high yield of xylitol. The yield is preferably above 60% more preferably above 70% and yields as high as 77% have been obtained. Xylitol can further be purified according to standard methods.

The present invention can be summarised as follows. The starting material is gluconic acid, whereby the acid is free, or in the form of its salt or the corresponding lactone. In the examples illustrating the present invention sodium gluconate and glucono-delta-lactone have been used as starting materials. Gluconic acid can be obtained for example through catalytic oxidation or through fermentation of glucose.

The gluconic acid is decarboxylated to arabinose according to methods known in the art. A well known method is the one described by Ruff in Berichte der Deutschen Chemischen Gesellschaft. 32 (1899) 553-554. An improved method for the production of arabinose is described in Hockett et al. J. Amer. Chem. Soc. 56 (1934) 1632-1633. A further method is described in Whistler et al. J. Amer. Chem. Soc. 81(1959) 5190-5192.

The conversion of gluconic acid to arabinose is performed in water and can for example be effectuated by the following two kinds of reagents: A) sodium hypochlorite or an organic hypohalite source e.g. N-chlorosuccinimide (in situ formation of hypochlorite) and B) hydrogen peroxide in the presence of catalytic amounts of Fe⁺⁺⁺ or Cu⁺⁺.

An advantage of the use of arabinose as an intermediate is that it can easily be purified by ion-exchange refining, this standard purification method would not be possible with arabinonic acid as an intermediate.

The obtained non-ionic arabinose is hydrogenated under mild reaction conditions with respect to hydrogen pressure and temperature and well known hydrogenation catalysts to give arabinitol. Suitable catalysts are ruthenium, especially supported ruthenium catalysts are used e.g. ruthenium-on-carbon, or nickel, especially Raney-nickel. The hydrogenation is routinely performed at temperatures between 70°C and 150°C and the pressure is between 0.1 and 10 MPa.

The obtained D-arabinitol is subjected to catalytic isomerisation.

D-arabinitol is treated at elevated temperatures, preferably above 100°C, and elevated pressures of hydrogen gas, preferably above 1 MPa, with hydrogenation/dehydrogenation catalysts such as ruthenium, copper, palladium, platinum, rhodium, cobalt and nickel based catalysts, and in general metal oxides and mixtures thereof. The polyol isomerisation is performed at distinctly different pH levels, and the addition of alkali or acid has an influence on the thermodynamic equilibrium of the pentitol mixture. The isomerisation reaction results in a product containing xylitol, ribitol and D,L-arabinitol. Xylitol is present in these mixtures in more than 10% preferably in more than 20%. This reaction product contains further some lower alditols, such as tetritols and triitols, adding up to maximum 10% preferably only to 5%.

The isomerisation mixture is optionally subjected to chromatography. It has been found that chromatography on cationic resin material gives xylitol with a purity in excess of 95%. Preferably the mixture is first demineralized and subsequently submitted to chromatography. The refining is suitably performed using a strong cation exchange resin e.g. Duolite 26 followed by a medium base anion exchange resin Duolite A 368. This process is preferable repeated. On plant scale chromatography is performed using suitable equipment obtainable for example from Mitsubishi with Diaion UBK-555 resin (in Ca²⁺ form). Separation methods have been extensively described for example in EP 0 403 392, and the references cited therein (page 5 line 39 - page 6 line 21).

The other pentitols are optionally recycled to the polyols isomerisation, resulting in an increased overall yield. The xylitol can also further be purified by crystallisation.

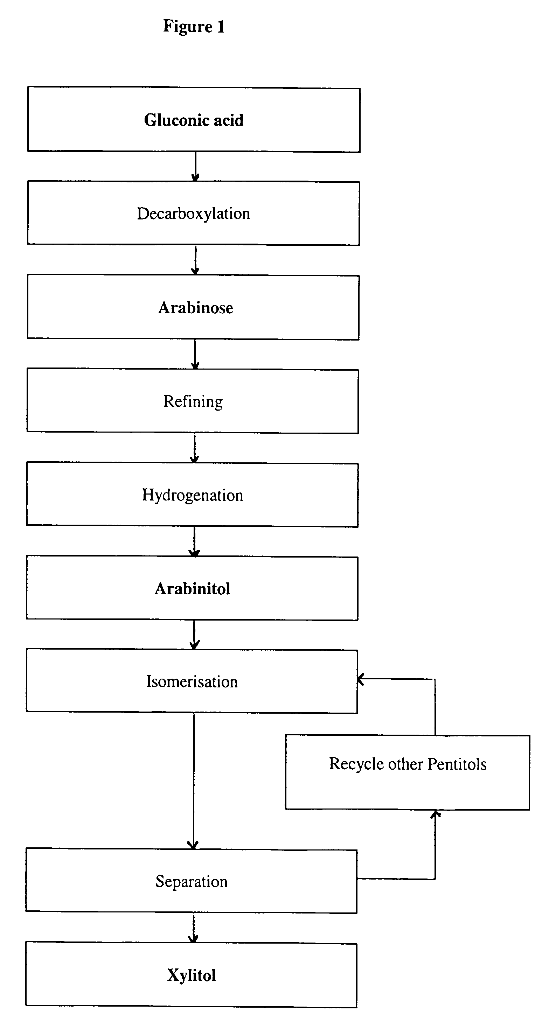

The advantage of this process in comparison with earlier described processes such as disclosed in WO93/19030 is that well established unit operations can be used for the refining of arabinose which would not be possible with arabinonic acid (classical syrup refining) and that known techniques and equipment for the hydrogenation (classical polyol hydrogenation) can be used. The advantage compared with methods such as those described in EP 403 392 and EP 421 882 is that although the reaction sequence up to arabinitol is also mentioned in these disclosures, the present invention gives a much shorter reaction sequence from arabinitol towards the desired pentitol; xylitol. Schematically the method of the present invention is illustrated in Figure 1.

The invention will be further illustrated in more detail in the following examples.

### Example 1

218 g Sodium gluconate (1 Mol) was dissolved in 800 ml demineralised water and the pH brought to 5.0. The solution was brought to 55°C and 1068 ml sodium hypochlorite (16% w/v) was added continuously over a period of 15 minutes, keeping the pH value between 4.9 and 5.1 with diluted hydrochloric acid. After all the hypochlorite had been added the reaction was allowed to continue for 30 minutes. No residual active chlorine was detectable after this period.

After demineralisation and refining, the product had the following composition: 96% D-arabinose, 2% D-glucose, 2 % unknown sugars (isomerisation products or C₄-sugars). The total weight yield of the above demineralised product was 137.5 g, of which 132 g was D-arabinose (88% of theory).

The arabinose syrup was hydrogenated on Raney Nickel (5% catalyst on total dry substance) applying a hydrogen pressure of 4 MPa at a temperature of 110°C. Hydrogenation was completed within 2.5 hours, the reducing sugar amount measured by DE measurement was lower than 0.1%.

Isomerisation of the formed D-arabinitol was performed by increasing the pH value in the hydrogenation autoclave to 9-10. After 6 hours at 170°C the reaction was terminated. The obtained demineralized isomerisate had the following pentitol composition: D,L-arabinitol (71%), ribitol (13%), xylitol (16%).

The xylitol was separated by chromatography on cation exchange resin in the calcium form, yielding xylitol with a purity of greater than 95%. The arabinitol and ribitol were recycled to isomerisation.

The obtained xylitol was crystallized.

### Example 2

178 g glucono-delta-lactone (1 Mol) was dissolved in 800 ml demineralised water and the pH brought to 5.5. As catalyst 5.4 g ferric sulphate was added. The solution was brought to 65°C and 204 ml hydrogen peroxide (30% w/v) was added in 4 portions with an interval of 1 hour, keeping the pH value between 5.4 and 5.6 by addition of diluted acetic acid. After all the hydrogen peroxide had been added the reaction was allowed to continue for 1 hour. No residual hydrogen peroxide was detectable after this period.

After demineralisation and refining the product had the following composition: 82% D-arabinose, 6% D-glucose, 12% unknown sugars (isomerisation products or C₄-sugars). The total weight yield of above demineralised product was 128 g, of which 105 g was D-arabinose (70% of theory).

The arabinose syrup was hydrogenated on a Ruthenium catalyst (2% catalyst on total dry substance), which was supported on active carbon (5% Ru on carbon). To the arabinose syrup phosphoric acid (1% on total dry substance) was added. The reaction temperature was 150°C at a hydrogen pressure of 4 MPa. Within 2 hours the residual reducing sugar content measured by DE measurement was lower than 0.1% and the isomerisation proceeded to a sufficient level. The addition of phosphoric acid induces isomerisation during the hydrogenation. The obtained hydrogenated syrup had the following composition. 81% total pentitols (of which 21% xylitol, 14% ribitol, 65% D,L-arabinitol) and 19 % tetritols and hexitols.

The xylitol was recovered as in example 1.

### Example 3

178 g glucono-delta-lactone (1 Mol) was dissolved in 800 ml demineralised water and the pH brought to 7.5. As catalyst 3.4 g copper(II) sulphate was added. The solution was brought to 65°C and 362.6 ml hydrogen peroxide (30% w/v) was added in 7 portions with an interval of 1 hour, keeping the pH value between 7.4 and 7.6 by addition of diluted sodium hydroxide. After all the hydrogen peroxide had been added the reaction was allowed to continue for 1 hour. No residual hydrogen peroxide was detectable after this period.

After demineralisation and refining the product had the following composition: 85% D-arabinose, 8% D-glucose, 7% unknown sugars (isomerisation products or C₄-sugars). The total weight yield of above demineralised product was 137 g, of which 117 g was D-arabinose (78% of theory).

The arabinose syrup was hydrogenated on a Ruthenium catalyst (4% catalyst on total dry substance), which was supported on active carbon (5% Ru on carbon). The reaction temperature was 135°C at a hydrogen pressure of 4 MPa. Within 1 hour the residual reducing sugar content measured by DE measurement was lower than 0.1%. The hydrogenated syrup had the following composition: 84% D-arabinitol and 16% tetritols and hexitols. To this hydrogenated syrup phosphoric acid was added (1% on total dry substance) and isomerisation was performed at 40 bar hydrogen pressure at 150°C for 3 hours. The obtained isomerised, hydrogenated syrup had the following composition: 83% total pentitols (of which 29% xylitol, 21% ribitol, 50% D,L-arabinitol) and 17 % tetritols and hexitols.

The xylitol was recovered as in example 1.

## Claims

1. A method of producing xylitol from gluconic acid wherein the acid is in free form or in the form of a salt or the corresponding lactone characterised in that the method comprises the following steps:
a. decarboxylation of gluconic acid, performed using (i) a hypochlorite or (ii) hydrogen peroxide in the presence of a catalytic amount of Fe³⁺ or Cu²⁺, wherein in both (i) and (ii) the pH is kept substantially constant, to give an intermediate consisting mainly of arabinose,
b. hydrogenation of the arabinose in the presence of a catalyst to give the corresponding pentitol, arabinitol,
c. catalytic isomerisation of the arabinitol to a pentitol mixture,
d. separation of xylitol from the pentitol mixture, and
e. optionally recycling of the other pentitols, mainly arabinitol and ribitol, to the isomerisation step (c).

2. A method according to claim 1 characterised in that the hydrogenation is performed in the presence of Ruthenium or Raney-Nickel as a catalyst.

3. A method according to claim 1 or claim 2 characterised in that the isomerisation is performed in the presence of a hydrogenation/dehydrogenation catalyst promoted by the addition of an alkali or an acid.

4. A method according to any one of the previous claims characterised in that the separation of xylitol is performed using a cationic resin.

5. A method according to any one of the previous claims characterised in that the xylitol is crystallised.

## Patentansprüche

1. Verfahren zur Herstellung von Xylit aus Gluconsäure, wobei die Säure in freier Form oder in Form eines Salzes oder des entsprechenden Lactons vorliegt, dadurch gekennzeichnet, daß das Verfahren die nachstehenden Schritte umfaßt:
a. Decarboxylierung von Gluconsäure, durchgeführt unter Verwendung von (i) einem Hypochlorit oder (ii) Wasserstoffperoxid in Gegenwart einer katalytischen Menge Fe³⁺ oder Cu²⁺, wobei sowohl in (i) als auch (ii) der pH-Wert im wesentlichen konstant gehalten wird, unter Gewinnung eines Zwischenprodukts, das hauptsächlich aus Arabinose besteht,
b. Hydrierung der Arabinose in Gegenwart eines Katalysators, unter Gewinnung des entsprechenden Pentits Arabit,
c. katalytische Isomerisierung des Arabits zu einem Pentitgemisch,
d. Abtrennung von Xylit aus dem Pentitgemisch und
e. gegebenenfalls Zurückführen der anderen Pentite, hauptsächlich Arabit und Ribit, zu dem Isomerisierungsschritt (c) .

2. Verfahren nach Anspruch 1, dadurch gekennzeichnet, daß die Hydrierung in Gegenwart von Ruthenium oder Raney-Nickel als Katalysator durchgeführt wird.

3. Verfahren nach Anspruch 1 oder Anspruch 2, dadurch gekennzeichnet, daß die Isomerisierung in Gegenwart eines Hydrierungs-/Dehydrierungskatalysators, gefördert durch die Zugabe eines alkalischen Stoffes oder einer Säure, durchgeführt wird.

4. Verfahren nach einem der vorangehenden Ansprüche, dadurch gekennzeichnet, daß die Abtrennung von Xylit unter Verwendung eines kationischen Harzes durchgeführt wird.

5. Verfahren nach einem der vorangehenden Ansprüche, dadurch gekennzeichnet, daß das Xylit kristallisiert wird.

## Revendications

1. Procédé de production de xylitol à partir d'acide gluconique dans lequel l'acide est sous forme libre ou sous la forme d'un sel ou de la lactone correspondante, caractérisé en ce que le procédé comprend les étapes suivantes :
a. la décarboxylation d'acide gluconique, effectuée en utilisant (i) un hypochlorite ou (ii) du peroxyde d'hydrogène en présence d'une quantité catalytique de Fe³⁺ ou de Cu²⁺, dans laquelle à la fois aux points (i) et (ii) le pH est maintenu sensiblement constant, afin d'obtenir un produit intermédiaire principalement composé d'arabinose,
b. l'hydrogénation de l'arabinose en présence d'un catalyseur afin d'obtenir le pentitol correspondant, l'arabinitol,
c. l'isomérisation catalytique de l'arabinitol en un mélange de pentitols,
d. la séparation du xylitol d'avec le mélange de pentitols, et
e. le cas échéant, le recyclage des autres pentitols, principalement l'arabinitol et le ribitol, vers l'étape d'isomérisation (c).

2. Procédé selon la revendication 1, caractérisé en ce que l'hydrogénation est effectuée en présence de ruthénium ou de nickel de Raney en tant que catalyseur.

3. Procédé selon la revendication 1 ou la revendication 2, caractérisé en ce que l'isomérisation est effectuée en présence d'un catalyseur d'hydrogénation/déshydrogénation activé par l'ajout d'un alcali ou d'un acide.

4. Procédé selon l'une quelconque des revendications précédentes, caractérisé en ce que la séparation du xylitol est effectuée au moyen d'une résine cationique.

5. Procédé selon l'une quelconque des revendications précédentes, caractérisé en ce que le xylitol est cristallisé.
